# EUROPÄISCHE PATENTANMELDUNG

(11) **EP 0 649 855 A1**
(43) Veröffentlichungstag der Anmeldung: **26.04.1995**
(21) Anmeldenummer: 94116219.0
(22) Anmeldetag: 14.10.1994
(51) Int. Cl.: C07H 19/04, C07H 21/00

(54) **Verwendung von mit enzymatisch abspaltbaren Schutzgruppen versehenen Nukleosiden und Nukleosid-Derivaten in der Synthese von Oligonukleotiden**

(30) Priorität: 20.10.1993 DE 4335729
(71) Anmelder: BOEHRINGER MANNHEIM GMBH, D-68298 Mannheim (DE)
(72) Erfinder: Waldmann, Herbert, Prof. Dr., D-76764 Rheinzabern (DE); Reidel, Armin, D-55411 Bingen (DE); Heuser, Axel, D-76137 Karlsruhe (DE); Mühlegger, Klaus, D-82398 Polling (DE); von der Eltz, Herbert, Dr., D-82362 Weilheim (DE); Birkner, Christian, Dr., D-82449 Uffing (DE)

(57) **Zusammenfassung**

Verfahren zur Herstellung von Oligonukleotiden der Formel II, in denen die exocyclischen Aminogruppen der Basen Adenin, Guanin, Cytosin, 7-Desaza-adenin und 7-Desaza-guanin N-Phenylacetylgruppen tragen, zur Oligonukleotidsynthese verwendet werden, wobei in einem ersten Schritt ein Startnukleotid an einen festen Träger gebunden wird, anschließend durch schrittweise Kupplung mit entsprechend aktivierten weiteren monomeren Nukleotidbausteinen der allgemeinen Formel I mit den obengenannten Bedeutungen das gewünschte Oligonukleotid aufgebaut wird, ggf. während und nach der Synthese dreiwertiger Phosphor zu fünfwertigem Phosphor oxidiert wird, das Oligonukleotid vom Träger und die 5'-Schutzgruppen abgespalten werden. Die Phenylacetylfunktionen, welche exocyclische NH₂-Gruppen der Basen schützen, können in schonender Weise mit Penicillinamidohydrolase (EC 3.5.1.11) abgespalten werden.

## Beschreibung

Die Erfindung betrifft Nukleotide und Nukleoside, deren exocyclische Aminogruppen durch Phenylacetatgruppen geschützt sind, sowie die Verwendung zur Herstellung von Oligonukleotiden.

Nukleinsäurederivate, wie Nukleoside und deren Phosphorsäureester, die Nukleotide, spielen eine wichtige Rolle in der Natur. Sie sind dort als Träger oder Überträger der genetischen Information von zentraler Bedeutung.

Mit zunehmender Kenntnis der diesen Vorgängen zugrunde liegenden molekularbiologischen Mechanismen ist es in den letzten Jahren möglich geworden, die Neukombination von Genen zu betreiben [siehe z. B. E.- L. Winnacker in "Gene und Klone, eine Einführung in die Gentechnologie", VCH Verlagsgesellschaft Weinheim (1985)]. Diese Technologie eröffnet neue Möglichkeiten in vielen Bereichen, z.B. der Medizin und der Pflanzenzüchtung.

Parallel zu der Erkenntnis molekularbiologischer Abläufe und Zusammenhänge hat sich vor allem in den vergangenen 10 Jahren die chemische Synthesetechnologie auf dem Gebiet der Nukleinsäuren sprunghaft fortentwickelt. Sie befruchtete ihrerseits wieder die oben genannte Entwicklung der Gentechnologie insgesamt. So ist es heute möglich, mit chemisch synthetisierten DNA-Bausteinen definierter Länge und Basensequenz (sogenannten Oligodesoxynukleotiden) unter Zuhilfenahme von Enzymen (Ligasen) ganze Gene künstlich herzustellen. In Form von sogenannten "Primern" dienen solche Oligomere als Startmoleküle für die enzymatische Synthese komplementärer, doppelsträngiger Nukleinsäuren an einem "template", d. h. einer einzelsträngigen Nukleinsäure mit Hilfe von Polymerasen. Dieser Technik bedient sich die zu außerordentlicher Bedeutung gekommene PCR-Methode als Möglichkeit der Amplifikation (Vermehrung) von DNA.

Das oben genannten Prinzip der geprimten Synthese von Nukleinsäuren macht sich auch die DNA-Sequenzierung zunutze. Die Kenntnis über die Sequenz, d. h. über die Basenabfolge bestimmter Gene oder Genbereiche des humanen Genoms z. B. bietet sowohl die Möglichkeit genetische Defekte diagnostisch zu detektieren, als auch zukünftig gezielt zu therapieren.

Als hochaktuelles diagnostisches Werkzeug kommen Oligonukleotide auch in Form sogenannter "Sonden" zum gezielten "Suchen" bakterieller bzw. viraler Infektionen zum Einsatz [Hames, Rickwood, Higgins (1985) in "Nucleic Acid Hybridisation: A Practical Approach" IRL Press].

Als eine die medizinische Therapie von vor allem viralen Infektionen möglicherweise revolutionierende Methode ist die sogenannte "Antisense"-Technologie in den letzten Jahren bekannt geworden [Uhlmann und Peyman (1990) in "Antisense Oligonucleotides: A New Therapeutic Principle", Chem. Rev. **90**, 543]. Dieser Technik liegt die Idee zugrunde, die genetische Information eines Virus' zu blockieren und damit die weitere Genexpression bzw. Virusreplikation zu verhindern. Diese Inhibierung kann durch zu viralen Genomabschnitten komplementäre Oligonukleotide bewerkstelligt werden, wobei durch Hybridisierung entweder die Messenger-RNA-Funktion blockiert wird, oder aber über die Ausbildung von Triplex-Strukturen eine Übertragung der genetischen Information verhindert wird.

Aus diesen beispielhaften Schilderungen ist die eminente Bedeutung der chemischen Synthese von Oligonukleotiden ersichtlich. Einen Überblick der Methoden gibt z.B. "Oligonucleotides and Analogues: A Practical Approach" (1991) (F. Eckstein, Hrsg.), IRL Press at Oxford University Press Oxford, New York, Tokyo.

Eines der zentralen Probleme dieser Synthesen liegt in der Struktur der monomeren Bausteine aller Nukleinsäuren begründet: der Multifunktionalität sowohl der heterocyclischen Basen, als auch der des Zuckerteils.

Die in allen Nukleinsäuren vorkommenden Nukleinsäurebasen Adenin, Guanin, Cytosin und Thymin bzw. Uracil tragen exocyclische Aminogruppen, die sämtlich in ungeschützter Form zu massiven Nebenreaktionen während der chemischen Synthese fuhren.

Deshalb müssen diese funktionellen Gruppen bei der Oligonukleotidsynthese mit geeigneten Schutzfunktionen versehen werden. Voraussetzung dafür sind schonende und vor allem selektive Bedingungen sowohl für die Einfuhrung dieser Schutzgruppen in die monomeren Synthesebausteine, als auch für die Abspaltung vom fertigen Oligomer nach beendeter Synthese. Üblicherweise werden diese Schutzgruppen nach der Synthese durch stark alkalische Bedingungen entfernt.

Die exocyclischen NH₂-Gruppen der heterocyclischen Basen Adenin, Cytosin und Guanin werden beispielsweise mit dem alkalilabilen Benzoyl- bzw. Isobutyryl-Rest geschützt. Daneben kommen in untergeordnetem Maße noch weitere Schutzgruppen wie Dimethylamino-methyliden- und Phenoxyacetylgruppen zum Einsatz.

Die Verwendung der Phenylacetyl-Funktion zum Schutz exocyclischer NH₂-Gruppen wird z. B. von Reese und Skone [J. Chem. Soc. Perkin Trans. I, 1263 (1984)] oder Moon und Huh [Bull. Korean Chem. Soc. **12,** 196 (1991)] beschrieben. Die Entfernung dieser Phenylacetyl-Schutzgruppen erfolgt mit in der Regel konz. Ammoniaklösung bei höherer Temperatur (50 ^{o}C) über mehrere Stunden. Dies fuhrt zu Nebenreaktionen; insbesonders bei Oligoribonukleotiden wird hier die Spaltung der Internukleotidbindung beobachtet. Besonders nachteilig kann sich die Verwendung alkalischer Abspaltbedingungen auf die Stabilität der Oligonukleotide und insbesondere oligonukleotidgebundener Gruppen, wie Reportergruppen wie z. B. Digoxigenin, auswirken. Solche Signalgruppen finden in den eingangs erwähnten Oligonukleotid-Sonden für diagnostische oder zellbiologische Fragestellungen allgemeinen Einsatz.

Die Aufgabe der Erfindung lag daher darin, an exocyclischen NH₂-Gruppen geschützte Nukleotide bereitzustellen, mit denen auf einfache und schonende Weise Oligonukleotide synthetisiert werden können.

Diese Aufgabe wird gelöst durch Nukleotide der allgemeinen Formel I, 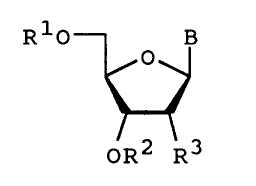
in denen B = Adenin, Guanin, Cytosin, 7-Desaza-adenin oder 7-Desaza-guanin bedeutet, R¹ = H oder eine Acyl-Schutzgruppe mit 1-4 C-Atomen oder eine 4,4'-Dimethoxy-trityl-Schutzgruppe, R² = H, eine Acyl-Schutzgruppe mit 1-4 C-Atomen, einen Phosphoramidit- oder Phosphonat-Rest oder eine an einen festen Träger gebundene Gruppe, R³ = H, OH oder OR' darstellt, wobei R' Acyl, Alkyl oder Alkenyl mit je 1-4 C-Atomen, oder eine Silylschutzgruppe bedeutet und die exocyclischen Aminogruppen der Basen B mit N-Phenylacetylgruppen geschützt sind.

Bevorzugt stellt R¹ eine Acetylgruppe dar, R² ein N,N-Dialkylamino-O-(2-cyanoalkyl)-phosphan, ein H-Phosphonat und als festen Träger Controlled Pore Glass oder Polystyrol.

Mit diesen Nukleotiden können in üblicher Weise Oligonukleotide synthetisiert werden. Die Durchführung von Oligonukleotidsynthesen ist dem Fachmann allgemein bekannt und beispielsweise in Gait, M.J. in "Oligonukleotide synthesis, a practical approach", IRL Press, LTD. 1984, Narang, S.A., "Synthesis and application of DNA and RNA", Academic Press 1987, beschrieben.

Ein weiterer Gegenstand der Erfindung sind Oligonukleotide der allgemeinen Formel II, 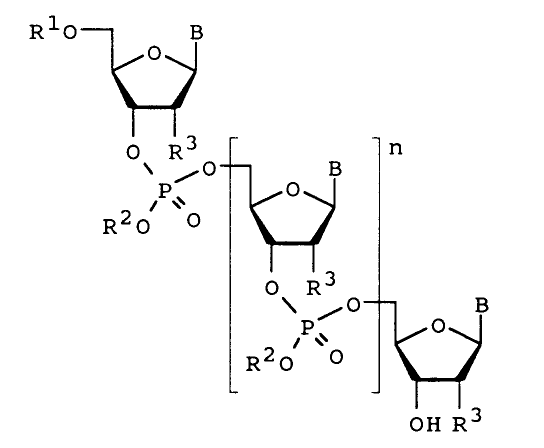
worin B Nukleinsäurebasen darstellen, R¹ = H oder eine Acyl-Schutzgruppe mit 1-4 C-Atomen oder eine 4,4'-Dimethoxy-trityl-Schutzgruppe, und R³ = H, OH oder OR', wobei R' vorzugsweise Acyl, Alkyl oder Alkenyl mit je 1-4 C-Atomen, oder eine Silylschutzgruppe bedeutet, R² = H, ein Alkaliatom oder eine Schutzgruppe und n eine Zahl zwischen 3 und 100, vorzugsweise zwischen 6 und 40 bedeutet und in denen die exocyclischen Aminogruppen der Basen Adenin, Guanin, Cytosin, 7-Desaza-adenin und 7-Desaza-guanin durch N-Phenylacetylgruppen geschützt sind.

Unter Nukleinsäurebasen sind die dem Fachmann allgemein bekannten Basen wie z.B. A T G C zu verstehen, in denen die Nukleinsäureketten üblicherweise in 1'-Stellung des Zuckers N-glycosidisch gebunden sind.

Zusätzlich können diese Oligonukleotide Phosphorthioat-Internukleotidbrücken enthalten. Solche Oligonukleotide haben ein hohes Potential in der therapeutischen Anwendung als sogenannte "Antisense" - Oligo-nukleotide, da sie äußerst resistent gegen den nukleolytischen Abbau durch zelluläre Nucleasen sind. Eine dem Fachmann bekannte Möglichkeit zur Herstellung bietet die "Oxidation" mit Schwefel oder anderen bekannten Sulfurierungsreagentien wie z. B. Tetraethyl-thiuramdisulfid.

In einer bevorzugten Ausführungsform werden die Oligonukleotide mit nachweisbaren Markierungen (Signalgruppe, Reportergruppe) versehen. Die radioaktive Markierung wird üblicherweise mit geeigneten Isotopen, wie ³²P oder ³⁵S durchgeführt.

Als nicht-radioaktive Indikatormoleküle haben sich u. a. hauptsächlich Haptene (wie Biotin oder Digoxigenin), Enzyme (wie alkalische Phosphatase oder Peroxidase), Lumiphore oder Fluoreszenzfarbstoffe (wie Fluorescein oder Rhodamin) bewährt (siehe z. B. "Nonradioactive Labeling and Detection of Biomolecules", C. Kessler (Hrsg.) Springer Verlag Berlin, Heidelberg 1992).

Signalmoleküle können über reaktive Funktionen sowohl am Oligonukleotid-, als auch am jeweiligen Signalmolekül kovalent an Oligonukleotide gebunden werden. Beispielsweise erfolgt die Reaktion von N-Hydroxysuccinimidestern von Haptenen oder Fluorophoren mit 5'-terminalen Aminofunktionen der Oligomeren.

In einer besonders bevorzugten Ausführungsform werden die Signalgruppen als letzten Schritt in der Oligonukleotidsynthese unter Verwendung von Phosphoramitiden am automatischen Synthesegerät eingeführt (Sinha, N.D., in Eckstein F., loc. cit., S. 185 ff).

Ein weiterer Gegenstand der Erfindung ist ein Verfahren zur Herstellung von Oligonukleotiden der Formel II, 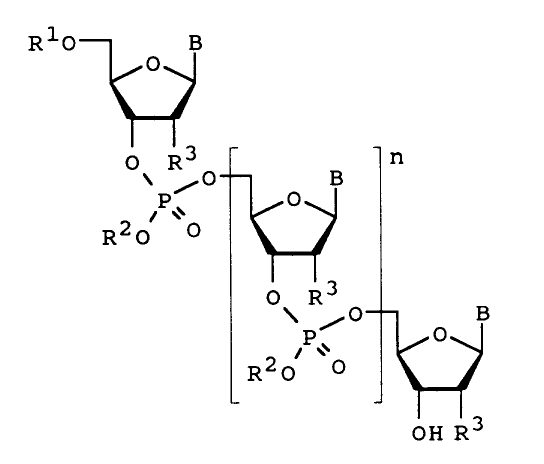
in der B Nukleinsäurebasen darstellen, R¹ = H oder eine Acyl-Schutzgruppe mit 1-4 C-Atomen oder eine 4,4'-Dimethoxy-trityl-Schutzgruppe, R² H, ein Alkaliatom oder eine Schutzgruppe, R³ = H, OH oder OR' darstellt, wobei R' vorzugsweise Acyl, Alkyl oder Alkenyl mit je 1-4 C-Atomen, oder eine Silylschutzgruppe bedeutet, welches dadurch gekennzeichnet ist, daß die Nukleotide der Formel I mit den dort angegebenen Bedeutungen und in denen die exocyclischen Aminogruppen der Basen Adenin, Guanin, Cytosin, 7-Desaza-adenin und 7-Desaza-guanin N-Phenylacetylgruppen tragen, zur Oligonukleotidsynthese verwendet werden und in einem ersten Schritt ein Startnukleotid an einen festen Träger gebunden wird, anschließend durch schrittweise Kupplung mit entsprechend aktivierten weiteren monomeren Nukleotidbausteinen der allgemeinen Formel I mit den obengenannten Bedeutungen das gewünschte Oligonukleotid aufgebaut wird, ggf. während und nach der Synthese dreiwertiger Phosphor zu fünfwertigem Phosphor oxidiert wird, das Oligonukleotid vom Träger und die 5'-Schutzgruppen abgespalten werden und die Phenylacetylgruppen durch Inkubation mit Penicillinamidohydrolase (EG 3.5.1.11) abgespalten werden.

Dazu werden zuerst die monomeren Synthesebausteine der Basen hergestellt, worin die reaktionsfähigen exocyclischen Aminogruppen des Adenins, Guanins und Cytosins mit der Phenylacetyl-Schutzgruppe versehen werden. Die anschließende Oligonukleotidsynthese geschieht in an sich bekannter Weise an einem festen Träger nach der Phosphat- oder der Phosphittriester-Methode oder nach dem H-Phosphonat-Verfahren. Bevorzugt werden die beiden letzteren Verfahren eingesetzt, wobei die Synthese üblicherweise mit automatisch arbeitenden Synthesegeräten erfolgt.

Dabei werden die mit den entsprechenden Schutzgruppen versehenen reaktiven Nukleotid-Bausteine (vorzugsweise Nukleosid-phosphoramidite) im Prinzip - abhängig von der gewünschten Basenabfolge (Sequenz) - in wiederkehrenden Cyclen fortlaufend an einen mit einem Startnukleosid versehenen festen Träger gekuppelt. Solche Träger sind vorzugsweise von anorganischer Natur, wie z. B. Controlled Pore Glass (CPG) oder aber organische Polymere wie z. B. Polystyrol. Das Trägermaterial trägt meist über einen mehr oder weniger langen, in der Regel mehr oder weniger alkalilabilen Spacer das jeweilige Startnukleosid. Die Synthese läuft im allgemeinen vom 3'- zum 5'-Ende des Oligonukleotides. Vor jeder Verknüpfung wird durch einen Säureschritt die 5'-OH-Schutzgruppe (vorzugsweise 4,4'-Dimethoxytrityl) entfernt. Gegebenenfalls wird während oder nach der Synthese 3-wertiger Phosphor zu 5-wertigem oxidiert und nicht-umgesetztes Nukleosid durch 5'-O-Acetylierung geschützt ("capping").

Nach beendeter Synthese wird das Produkt durch kurzzeitige Behandlung mit Alkali oder organischen Basen vom festen Träger abgespalten. Die Entfernung der Phenylacetyl-Schutzgruppen von den exocyclischen Amino-Funktionen erfolgt enzymatisch mittels Penicillin-amidohydrolase (Pen-amidase, EC 3.5.1.11). Die Umsetzung erfolgt vorzugsweise unter milden alkalischen Bedingungen bei pH 7 bis 10, besonders bevorzugt bei pH-Werten um pH 8, über mehrere Stunden. Bei Raumtemperatur wird nach ca. 3 bis 4 Stunden eine Umsetzungsrate von ca. 85 bis 90 % erreicht. Die verwendete Menge an Pen-amidase ist an sich unkritisch. Es ist jedoch vorteilhalt, pro mMol Oligonukleotid ca. 5 bis 100 Enzymeinheiten Pen-amidase einzusetzen.

In einer weiteren Ausführungsform kann die primäre CH₂OH-Funktion am Zuckerteil mit der sehr säurelabilen Dimethoxy-trityl(DMTR)-Schutzgruppe, die 2'-OH-Gruppe in Oligoribonukleotiden mit der t-Butyl-dimethyl-silyl-Funktion geschützt werden.

In einer bevorzugten Ausführungsform wird die säurelabile Dimethoxytrityl-Schutzfunktion der 5'-OH-Gruppierung durch eine enzymatisch abspaltbare Schutzgruppe, besonders bevorzugt durch Acetyl (abspaltbar mit Acetylesterase, EC 3.1.1.6) ersetzt. Überraschenderweise läßt sich aus 3',5'-O-Diacetyl-N-phenylacetylierten Nukleosiden die 5'-O-Acetylgruppe selektiv mit Acetylesterase abspalten unter Erhalt der 3'-O-Acetyl-Schutzgruppe. Liegen die Nukleoside nur in 3',5'-O-Diacetyl-Form und nicht in N-phenylacetylierter Form vor, so spaltet Acetylesterase selektiv nur die 3'-O-Acetylfunktion ab und die 5'-O-Acetyl-Schutzgruppe bleibt erhalten. Die Herstellung solcher partiell geschützter Nukleoside ist von Bedeutung für zahlreiche einschlägige Synthesen in der Nukleinsäurechemie.

Die folgenden Beispiele erläutern die Erfindung näher:

### Beispiel 1:

### 3',5'-Di-O-acetyl-2'-desoxy-adenosin

1 g 2'-Desoxy-adenosin (3,8 mmol) werden in einem Gemisch aus 50 ml Acetonitril und 1,4 ml Triethylamin (10 mmol) suspendiert, der Suspension 75 mg 4-Dimethylaminopyridin und unter weiterem Rühren 1 ml Essigsäureanhydrid (10,5 mmol) zugefügt. Man rührt über Nacht bei Raumtemperatur, gibt dann 1 ml Methanol zu der Reaktionslösung und rührt weitere 10 Minuten. Man filtriert den Feststoff ab, wäscht mit Ethanol und Diethylether und trocknet im Vakuum.
Ausbeute: 1,17 g = 92 % d. Th.
DC (Kieselgel; Methanol:Ethylacetat = 1:2) : R_{f}0,51
¹H-NMR (D₂O): 1,98 und 2,16 (3H, s, 2xCH₃), 2,76 (1H, m, H_{2''}), 3,00 (1H, m, H_{2'}), 4,32 (2H, m, H_{5'/5''}), 4,50 (1H, m, H_{4'}), 5,50 (1H, m, H_{3'}), 6,44 (1H, dd, H_{1'}), 8,21 (1H, s, H₂), 8,30 (1H, s, H₈).

In derselben Weise wird **3',5'-Di-O-acetyl-2'-desoxy-guanosin** hergestellt. Es werden 1,37 g des Derivates in praktisch quantitativer Ausbeute erhalten.
DC (Kieselgel; Methanol:Ethylacetat = 1:2) : R_{f}0,47
¹H-NMR (DMSO-d₆): 1,94 und 1,97 (3H, s, 2xCH₃), 2,33 (1H, m, H_{2''}), 2,81 (1H, m, H_{2'}),
4,11 (2H, m, H_{4'/5''}), 4,15 (1H, m, H_{5'}), 5,19 (1H, m, H_{3'}), 6,03 (1H, dd, H_{1'}), 6,40 (2H, bs, NH₂), 7,80 (1H, s, H₈).

### Beispiel 2:

### 3',5'-Di-O-acetyl-N⁶-phenylacetyl-2'-desoxy-adenosin

Zu 1 g 3',5'-Di-O-acetyl-2'-desoxy-adenosin (3 mmol) wird eine Lösung von 3 g Phenylessigsäureanhydrid (12 mmol) in 15 ml wasserfreiem Pyridin gegeben und 2 Stunden bei 120 ^{o}C gerührt. Nach Abkühlen auf Raumtemperatur wird das Reaktionsgemisch unter Rühren in 40 ml einer gesättigten NaHCO₃-Lösung gegossen. Man extrahiert 6x mit je 20 ml Dichlormethan und trocknet die vereinigten organischen Phasen über MgSO₄. Nach Filtrieren und Einengen im Vakuum erhält man eine braunes Öl, welches mittels Säulenchromatographie an Kieselgel (Laufmittel CHCl₃/Ethanol, 20:1,v/v) aufgereinigt wird. Die entsprechenden reinen Fraktionen werden vereinigt und eingedampft.
Ausbeute: 1,18 g = 87 % d. Th.
DC (Kieselgel; Methanol:Ethylacetat = 1:2) : R_{f}0, 64
¹H-NMR (CDCl₃): 2,01 und 2,08 (3H, s, 2xCH₃), 2,58 (1H, m, H_{2''}), 2,90 (1H, m, H_{2'}), 4,14 (2H, s, CH₂-Phe), 4,30 (3H, m, H_{4'/}H_{5'/5''}), 5,38 (1H, m, H_{3'}), 6,40 (1H, dd, H_{1'}), 7,33 (5H, m, CH₂-Phe), 8,11 (1H, s, H₂), 8,63 (1H, s, H₈), 8,77 (1H, bs, NH).

Nach dem gleichen Verfahren wird **3',5'-Di-O-acetyl-N²-phenylacetyl-2'-desoxy-guanosin** erhalten.
Ausbeute: 1,05 g = 85 % d. Th.
DC (Kieselgel; Methanol:Ethylacetat = 1:2) : R_{f}0,59
¹H-NMR (CDCl₃): 2,08 und 2,11 (3H, s, 2xCH₃), 2,49 (1H, m, H_{2''}), 2,91 (1H, m, H_{2'}), 3,74 (2H, s, CH₂-Phe), 4,37 (2H, m, H_{5'/5''}), 4,63 (1H, m, H_{4'}), 5,38 (1H, m, H_{3'}), 6,17 (1H, dd, H_{1'}), 7,36 (5H, m, CH₂-Phe), 7,72 (1H, s, H₈), 9,17 (1H, bs, NH-CO), 11,93 (1H, bs, N¹-H).

### Beispiel 3:

### Enzymatische Hydrolyse der N²-Phenylacetyl-Schutzgruppe aus 3',5'-Di-O-acetyl-N²-phenylacetyl-2'-desoxy-guanosin

0,95 g 3',5'-Di-O-acetyl-N²-phenylacetyl-2'-desoxy-guanosin (2 mmol) werden in 25 ml Methanol gelöst und zu 75 ml einer 0,07 m Phosphatpufferlösung, pH 7,5 gegeben. Man stellt mit 0,1 n NaOH auf pH 8,0 ein und gibt dann 100 Einheiten Penicillin-G-Amidase (EC 3.5.1.11) zu. Mit einem pH-Stat-Gerät wird der pH-Wert durch Titration mit 0,1 n NaOH in der Folgezeit auf 8,0 gehalten und über einen Schreiber protokolliert. Die Umsatzrate beträgt etwa 85 %. Nach Extraktion mit Dichlormethan und Eindampfen werden 460 mg 3',5'-Di-O-acetyl-2'-desoxy-guanosin, entsprechend 65 % der Theorie erhalten.

Die ¹H-NMR Daten entsprechen denen aus Beispiel 1.

Nach dem gleichen Verfahren werden die Derivate **3',5'-Di-O-acetyl-2'-desoxy-adenosin und -cytidin** gewonnen.

### Beispiel 4

### Selektive enzymatische Abspaltung der 5'-O-Acetyl-Schutzgruppe aus 3',5'-Di-O-acetyl-N²-phenylacetyl-2'-desoxy-guanosin mittels Acetyl-Esterase

0,5 mmol des nach Beispiel 2 erhaltenen diacetylierten Nukleosides werden in 350 ml 0,15 N NaCl-Lösung durch mehrstündiges Rühren gelöst. Eine Behandlung mit Ultraschall beschleunigt dies. Man stellt den pH-Wert auf 6,5 und fügt 10 Einheiten Acetyl-Esterase (EC 3.1.1.6) zu. Der pH wird durch Zutitrieren von 0,02 N NaOH am Combititrator konstant gehalten. Nach vollständiger Umsetzung (Kontrolle mittels DC aufKieselgel, Fließmittel Chloroform/Methanol 80:20) wird die Reaktionslösung mit Dichlormethan 3x ausgeschüttelt, die vereinigten organischen Phasen über Na₂SO₄ getrocknet und i.V. einkonzentriert. Man gibt das Konzentrat auf eine Kieselgel-60-Säule und eluiert das gewünschte Produkt mit Chloroform/Methanol 80:20. Die Produktfraktionen werden vereinigt und zum Öl eingeengt. Man nimmt in 25 ml Dioxan aufund lyophilisiert. Das 3'-O-Acetyl-N²-phenylacetyl-2'-desoxyguanosin wird als fast weißes amorphes Pulver in 47 % Ausbeute erhalten.
¹H-NMR (DMSO-d₆): 2,05 (3H, s, CO-CH₃), 2,50 (1H, dd, H_{2'}), 2,85 (1H, m, H_{2''}), 3,32 (2H, m, H_{5'/5''}), 3,80 (2H, s, CH₂-Phe), 4,04 (1H, m, H_{4'}), 5,12 (1H, t, OH_{5'}), 5,31 (1H, dd, H_{3'}), 6,23 (1H, dd, H_{1'}), 7,33 (5H, m, CH₂-Phe), 8,27 (1H, s, H₈), 11,97 (2H, s, NH-CO).

In analoger Weise wird aus dem entsprechenden Desoxy-adenosin-Derivat in 41 % Ausbeute **3'-O-acetyl-N⁶-phenylacetyl-2'-desoxy-adenosin erhalten.**
¹H-NMR (DMSO-d₆): 2,03 (3H, s, CO-CH₃), 2,48 (1H, dd, H_{2'}), 2,95 (1H, m, H_{2''}), 3,53 (2H, m, H_{5'/5''}), 3,82 (2H, s, CH₂-Phe), 4,05 (1H, m, H_{4'}), 5,12 (1H, t, OH_{5'}), 5,35 (1H, dd, H_{3'}), 6,40 (1H, dd, H_{1'}), 7,30 (5H, m, CH₂-Phe), 8,55 (1H, s, H₈), 8,60 (1H, s, H₂), 10,92 (1H, s, NH).

### Beispiel 5

### Selektive enzymatische Abspaltung der 3'-O-acetyl-Schutzgruppe aus 3',5'-Di-O-acetyl-2'-desoxy-guanosin mittels Acetyl-Esterase

Nach dem Verfahrensprinzip aus Beispiel 4 wird in 31 %iger Ausbeute **5'-O-Acetyl-2'-desoxy-guanosin** erhalten.
¹H-NMR (DMSO-d₆): 2,07 (3H, s, CO-CH₃), 2,36 (1H, m, H_{2'}), 2,76 (1H, m, H_{2''}), 3,34 (1H, m, H_{4'}), 3,58 (2H, m, H_{5'/5''}), 4,01 (1H, m, H_{3'}), 5,29 (1H, d, OH_{5'}), 6,11 (1H, dd, H_{1'}), 6,68 (2H, bs, NH₂), 7,91 (1H, s, H₈), 11,12 (1H, bs, NH).

Ebenso wird aus 3',5'-Di-O-acetyl-2'-desoxy-adenosin **5'-O-Acetyl-2'-desoxy-adenosin** erhalten.
¹H-NMR (DMSO-d₆): 2,08 (3H, s, CO-CH₃), 2,42 (1H, dd, H_{2'}), 2,95 (1H, m, H_{2''}), 3,63 (2H, m, H_{5'/5''}), 4,08 (1H, dd, H_{4'}), 5,35 (1H, d, OH_{5'}), 5,52 (1H, dd, H_{3'}), 6,35 (1H, dd, H_{1'}), 7,38 (2H, s, NH₂), 8,13 (1H, s, H₂), 8,36 (1H, s, H₈).

### Beispiel 6

### 3',5'-O-(Tetraisopropyldisiloxan-1,3-diyl)-2'-desoxy-adenosin

Die Synthese dieser Verbindung erfolgte im Prinzip nach Markiewicz, J. Chem. Res. (S) **24** (1979), indem zu einer magnetisch gerührten Suspension von 1,08 g 2'-Desoxy-adenosin (4 mmol) in 12 ml wasserfreiem Pyridin 1,4 ml 1,3-Dichlor-1,1,3,3-tetraisopropyldisiloxan (4,4 mmol) gegeben und das Reaktionsgemisch 5 Stunden bei Raum-temperatur gerührt wurde. Die Umsetzung war nach DC auf Kieselgel im Gemisch CHCL₃-CH₃OH 9:1 vollständig. Nach Abziehen des Pyridins i. V. wurde der Rückstand mit je 100 ml CHCl₃ und gesättigter wässriger NaHCO₃-Lösung extrahiert. Die wäßrge Phase wurde zweimal mit je 50 ml CHCl₃ ausgeschüttelt, die organischen Phasen vereinigt, über Na₂SO₄ getrocknet und i. V. eingedampft. Nach Aufnehmen in CHCl₃ wurde auf eine kurze Silicagel-Säule gegeben und mit anfangs Chloroform, dann mit Chloroform-Methanol getrennt. Die sauberen Fraktionen wurden vereinigt und bis zum festen, schaumigen Rückstand eingeengt.
Ausbeute: 1,88 g = 95 % d. Th.
DC (Kieselgel; Chloroform-Ethanol 20:1): R_{f}0,36
¹H-NMR (CDCl₃): 1,06 (30H, m, (CH₃)₂CH), 2,68 (2H, m, H_{2'/2''}), 3,88 (1H, m, H_{4'}), 4,04 (2H, m, H_{5'/5''}), 4,94 (1H, m, H_{3'}), 5,80 (2H, bs, NH₂), 6,92 (1H, dd, H_{1'}), 8,03 (1H, s, H₂), 8,31 (1H, s, H₈).

Nach dem gleichen Verfahren werden **3',5'-O-(Tetraisopropyldisiloxan-1,3-diyl)-2'-desoxy-guanosin und 3',5'-O-(Tetraisopropyldisiloxan-1,3-diyl)-2'-desoxy-cytidin** erhalten.
Ausbeute an **dGuanosin**-Derivat: 1,32 g = 65 % d. Th.
DC (Kieselgel; Chloroform-Ethanol 20:1): R_{f}0,18
Ausbeute an **dCytidin**-Derivat: 1,06 g = 57 % d. Th.
DC (Kieselgel; Chloroform-Ethanol 20:1): R_{f}0,24

### Beispiel 7

### 3',5'-O-(Tetraisopropyldisiloxan-1,3 diyl)-N⁶-phenylacetyl-2'-desoxy-adenosin

Die Verbindung wurde nach Beispiel 2 durch Umsetzung von 0,99 g (2 mmol) des 3',5'-O-geschützten Nukleosides aus Beispiel 6 mit 2,03 g Phenylessigsäureanhydrid (8 mmol) in 15 ml wasserfreiem Pyridin erhalten. Chromatographie an Kieselgel H zunächst mit Dichlormethan, dann mit Chloroform.
Ausbeute: 0,856 g = 87 % d. Th.
DC (Kieselgel; Chloroform-Ethanol 20:1): R_{f}0,70
Elementaranalyse (C₃₀H₄₅N₅O₅Si₂): C_{ber} 58,93; H_{ber} 7,36; N_{ber} 11,45; C_{gef} 59,2; H_{gef} 7,55; N_{gef} 11,1
¹H-NMR (CDCl₃): 1,05 (28H, m, (CH₃)₂CH), 2,70 (2H, m, H_{2'/2''}), 3,89 (1H, m, H_{4'}), 4,03 (2H, d, H_{5'/5''}), 4,20 (2H, s, CH₂-Phe), 4,95 (1H, m, H_{3'}), 6,30 (1H, dd, H_{1'}), 7,33 (5H, m, CH₂-Phe), 8,17 (1H, s, H₂), 8,59 (1H, bs, NH), 8,66 (1H, s, H₈).

In analoger Weise erhält man das entsprechende **N²-phenylacetyl-2'-desoxy-guanosin- bzw. N⁴-phenylacetyl-2'-desoxy-cytidin-**Derivat.

### Beispiel 8

### N⁶-Phenylacetyl-2'-desoxy-adenosin

3 g (5 mmol) 3',5'-O-(Tetraisopropyldisiloxan-1,3 diyl)-N⁶-phenylacetyl-2'-desoxy-adenosin werden in 20 ml trockenem Tetrahydrofuran (THF) gelöst und 10 ml einer 1,1 M Tetrabutylammonium-fluorid-Lösung in THF unter Rühren zugefügt. Nach ca. 10 min zeigt das Dünnschicht-chromatogramm (Kieselgel, Ethanol/Chloroform 10:90) die praktisch vollständige Abspaltung der Silyl-Schutzgruppe (R_{f} ca. 0,2, Ausgangsmaterial ca. 0,5) an. Die Reaktion wird durch Zugabe von 25 ml einer Mischung aus Pyridin/Methanol/Wasser (3:1:1) gestoppt und 30 min bei RT gerührt. Die Lösung wird im Vakuum bis zum Öl eingedampft, in wenig Ethanol aufgenommen und auf eine Kieselgel-Säule gegeben. Nach Elution mit Chloroform/Ethanol 90:10 werden die Produkt-fraktionen vereinigt, über Na₂SO₄ getrocknet und einkonzentriert.
Ausbeute: 1,43 g = 80,1 % d. Th.

In analoger Weise wurden **N²-Phenylacetyl-2'-desoxy-guanosin- und N⁴-Phenylacetyl-2'-desoxy-cytidin** erhalten.

### Beispiel 9

### 5'-O-(4,4'-Dimethoxytrityl)-N⁶-phenylacetyl-2'-desoxy-adenosin

1,25 g N⁶-Phenylacetyl-2'-desoxy-adenosin (3,5 mmol) aus Beispiel 8 werden in 25 ml trockenem Pyridin gelöst und das Lösungsmittel i. V. abgezogen. Diese Prozedur wird 2 x wiederholt, dann löst man den Rückstand in 50 ml wasserfreiem Pyridin und fügt nacheinander 1,75 g
4,4'-Dimethoxytriphenylmethylchlorid (5,25 mmol) und 0,9 ml Diisopropyl-ethylamin (5,25 mmol) zu. Man rührt ca. 3 Stunden bei RT, gießt danach die Reaktionslösung in 100 ml 5 %iges wässriges NaHCO₃ ein und extrahiert 2x mit je 100 ml Dichlormethan. Nach Trocknung der vereinigten organischen Phasen über Na₂SO₄ wird eingedampft, 3x mit Toluol coevaporiert und letztlich an Kieselgel 60 mittels Flash-Chromatographie aufgereinigt (Säule 6x15 cm, Dichlormethan-Methanol 98:2, 1 % Triethylamin). Die Produktfraktionen werden vereinigt, getrocknet und eingeengt bis zum schwach gelblichen schaumigen Rückstand.
Ausbeute: 1,7 g = 73,9 %
DC (Kieselgel; Dichlormethan-Methanol 95:5): R_{f}0,75
In analogen Verfahren erhält man die **4,4'-Dimethoxytrityl-Derivate** des **N²-Phenylacetyl-2'-desoxy-guanosin** und **N⁴-Phenylacetyl-2'-desoxy-cytidin**.

### Beispiel 10

### 5'-O-(4,4'-Dimethoxytrityl)-N⁶-phenylacetyl-2'-desoxy-adenosin-3'-[2-(cyanoethyl)-N,N-diisopropyl-phosphoramidit]

Zu einer Lösung von 1,6 g 5'-O-(4,4'-Dimethoxytrityl)-N⁶-phenylacetyl-2'-desoxy-adenosin (2,5 mmol) und 1,3 ml N-Ethyl-diisopropylamin (7,5 mmol) in trockenem THF werden unter Rühren 57 µl Chlor-β-cyanoethoxy-(N,N-diisopropylamino)phosphan (2,55 mmol) während 2 Minuten unter Argon bei RT zugetropft. Man stoppt nach etwa 30 Minuten die Reaktion durch Zugabe von 50 ml 5%iger wässriger NaHCO₃-Lösung. Das Reaktionsgemisch wird 2x mit je 50 ml Dichlormethan extrahiert, die organischen Extrakte vereinigt und über Na₂SO₄ getrocknet. Nach Eindampfen und Flash-Chromatographie (Kieselgel 60; Ethylacetat-Dichlormethan-Et₃N 45:45:10) werden die reinen Fraktionen gesammelt, über MgSO₄ getrocknet und zum Öl einkonzentriert. Nach Lösen in 50 ml Dioxan und Lyophilisation erhält man das Diastereomeren-Gemisch als cremefarbenes Produkt.
Ausbeute: 1,70 g = 79 % d. Th.
DC (Kieselgel; Dichlormethan-EtOAc-Et₃N 45:45:10): R_{f}0,58 und 0,60
³¹P-NMR (CDCl₃): 149 ppm und 152 ppm
Die **Phosphoramidite** des **4,4'-Dimethoxytrityl-N²-phenylacetyl-2'-desoxy-guanosin** und **4,4'-Dimethoxytrityl-N⁴-phenylacetyl-2'-desoxy-cytidin** werden nach demselben Verfahren gewonnen. Das entsprechende **Thymidin-Derivat ist** kommerziell erhältlich.

### Beispiel 11

### Herstellung von 5'-O-(4,4'-Dimethoxytrityl)-N⁶-phenylacetyl-2'-desoxy-adenosin-beladenem Controlled Pore Glass (CPG)-Träger

Das geschützte Nukleosid aus Beispiel 9 wurde nach den in der Literatur zitierten Standard-Verfahren (z.B. Adams, S. P. et al. (1983) J. Am. Chem. Soc. **105**, 661-663) an CPG beads gebunden.

Nach demselben Verfahren wurden die CPG-Träger für **2'-Desoxy-guanosin** und **2'-Desoxy-cytidin** mit den nach Beispiel 9 erhaltenen vollgeschützten Nukleosiden hergestellt.

### Beispiel 12

### Festphasen-Synthese eines Oligodesoxynukleotides der Sequenz d(AATTCCGGAATT) unter Verwendung N-Phenylacetyl-geschützter Nukleosid-phosphoramidite

Die Synthese des Oligomeren erfolgte nach dem Standardprotokoll für das Phosphoramidit-Verfahren (Applied Biosystems Users Manual des DNA-Synthesizers 380 B) im 1 µmol-Maßstab. Nach üblicher Abspaltung des Oligonukleotides vom CPG-Trägermaterial erfolgte die Entfernung der N-Phenylacetyl-Schutzgruppen sehr schonend wie unter Beispiel 3 angegeben.

## Patentansprüche

1. Nukleotide der allgemeinen Formel I, 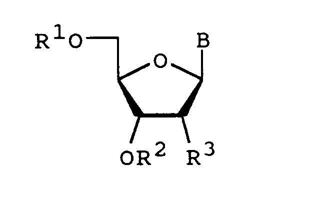 in denen
B = Adenin, Guanin, Cytosin, 7-Desaza-adenin oder 7-Desaza-guanin bedeutet,
R¹ = H oder eine Acyl-Schutzgruppe mit 1-4 C-Atomen oder eine 4,4'-Dimethoxy-trityl-Schutzgruppe,
R² = H, eine Acyl-Schutzgruppe mit 1-4 C-Atomen, einen Phosphoramidit- oder Phosphonat-Rest oder eine an einen festen Träger gebundene Gruppe,
R³ = H, OH oder OR' darstellt, wobei R' Acyl, Alkyl oder Alkenyl mit je 1-4 C-Atomen, oder eine Silylschutzgruppe bedeutet und die exocyclischen Aminogruppen der Basen B mit N-Phenylacetylgruppen geschützt sind.

2. Nukleotide nach Anspruch 1, in denen R¹ eine Acetylgruppe darstellt.

3. Nukleotide nach den Ansprüchen 1 bis 2, in denen R² ein N,N-Dialkylamino-O-(2-cyanoalkyl)phosphan darstellt.

4. Nukleotide nach den Ansprüchen 1 bis 3, in denen R² ein H-Phosphonat bedeutet.

5. Nukleotide nach den Ansprüchen 1 bis 4, in denen R² als fester Träger Controlled Pore Glass oder Polystyrol bedeutet.

6. Oligonukleotide der allgemeinen Formel II 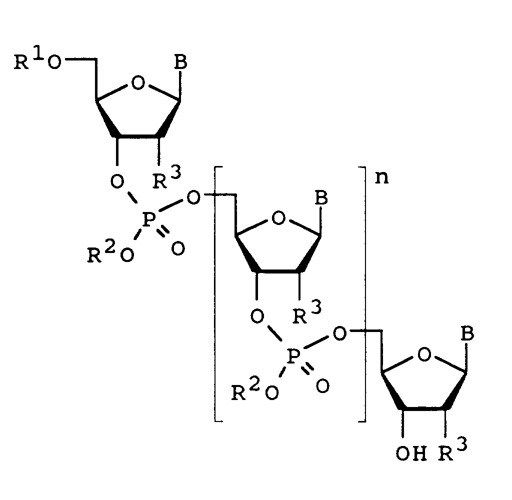 worin
B Nukleinsäurebasen darstellen,
R¹ = H oder eine Acyl-Schutzgruppe mit 1-4 C-Atomen oder eine 4,4'-Dimethoxy-trityl-Schutzgruppe
und R³ = H, OH oder OR', wobei R' vorzugsweise Acyl, Alkyl oder Alkenyl mit je 1-4 C-Atomen, oder eine Silylschutzgruppe bedeutet,
R² = H, ein Alkaliatom oder eine Schutzgruppe
und n eine Zahl zwischen 3 und 100, vorzugsweise zwischen 6 und 40 bedeutet
und in denen die exocyclischen Aminogruppen der Basen Adenin, Guanin, Cytosin, 7-Desaza-adenin und 7-Desaza-guanin durch N-Phenylacetylgruppen geschützt sind.

7. Oligonukleotide nach Anspruch 6, dadurch gekennzeichnet, daß zusätzlich eine Reportergruppe wie ein Hapten, ein Fluorophor oder ein Luminophor enthalten ist.

8. Verfahren zur Herstellung von Oligonukleotiden der Formel II, in der B Nukleinsäurebasen darstellt,
R¹ = H oder eine Acyl-Schutzgruppe mit 1-4 C-Atomen oder eine 4,4'-Dimethoxy-trityl-Schutzgruppe,
R² H, ein Alkaliatom oder eine Schutzgruppe darstellt,
R³ = H, OH oder OR' darstellt, wobei R' vorzugsweise Acyl, Alkyl oder Alkenyl mit je 1-4 C-Atomen, oder eine Silylschutzgruppe bedeutet, welches dadurch gekennzeichnet ist, daß die Nukleotide der Formel I 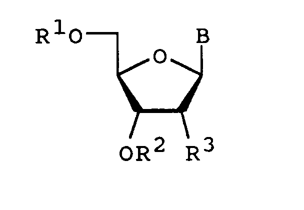 in denen
B = Adenin, Guanin, Cytosin, 7-Desaza-adenin oder 7-Desaza-guanin bedeutet,
R¹ = H oder eine Acyl-Schutzgruppe mit 1-4 C-Atomen oder eine 4,4'-Dimethoxy-trityl-Schutzgruppe,
R² = H, eine Acyl-Schutzgruppe mit 1-4 C-Atomen, einen Phosphoramidit- oder Phosphonat-Rest oder eine an einen festen Träger gebundene Gruppe,
R³ = H, OH oder OR' darstellt, wobei R' Acyl, Alkyl oder Alkenyl mit je 1-4 C-Atomen, oder eine Silylschutzgruppe bedeutet, in denen die exocyclischen Aminogruppen der Basen Adenin, Guanin, Cytosin, 7-Desaza-adenin und 7-Desaza-guanin N-Phenylacetylgruppen tragen, zur Oligonukleotidsynthese verwendet werden,
wobei in einem ersten Schritt ein Startnukleotid an einen festen Träger gebunden wird, anschließend durch schrittweise Kupplung mit entsprechend aktivierten weiteren monomeren Nukleotidbausteinen der allgemeinen Formel I mit den obengenannten Bedeutungen das gewünschte Oligonukleotid aufgebaut wird, ggf. während und nach der Synthese dreiwertiger Phosphor zu fünfwertigem Phosphor oxidiert wird, das Oligonukleotid vom Träger und die 5'-Schutzgruppe abgespalten werden und die Phenylacetylgruppen durch Inkubation mit Penicillinamidohydrolase (EC 3.5.1.11) abgespalten werden.

9. Verfahren nach Anspruch 8, dadurch gekennzeichnet daß von 3',5'-Diacetyl-N-phenylacetyl-nukleosiden ausgegangen und mit Acetylesterase (EC 3.1.1.6) der 5'-O-Acetyl-Rest abgespalten wird.
